# EUROPEAN PATENT APPLICATION

(11) **EP 2 388 325 A1**
(43) Date of publication of application: **23.11.2011**
(21) Application number: 10005298.4
(22) Date of filing: 20.05.2010
(51) Int. Cl.: C12N 15/10, G01N 33/53, C07K 14/01

(54) **Method for isolating small RNA-molecules using HC-Pro protein**

(71) Applicant: RLP AgroScience GmbH, 67435 Neustadt/Weinstrasse (DE)
(72) Inventor: Füllgrabe, Marc, 24601 Wankendorf (DE); Wassenegger, Michael, Dr., 67435 Neustadt (DE); KaJohn, Boonrod, Dr., 67433 Neustadt (DE); Krczal, Gabriele, Prof. Dr., 67433 Neustadt (DE)
(74) Representative: Krauss, Jan

(57) **Abstract**

The present invention relates to a method for isolating small RNA-molecules (sRNAs) from a sample using Helper-component-protein (HC-Pro) from potyviruses. The present invention furthermore relates to an improved method for isolating sRNAs from a sample using HC-Pro together with a cupin protein or the cupin 2-superfamily domain thereof. The methods of the present invention can be used in the treatment, diagnosis and monitoring of diseases, for screening compounds modifying the number, amount, and/or concentration of sRNAs. Finally, the invention also relates to a kit for isolating sRNAs from a sample.

SEQUENCE LISTING

<110>  RLP AgroScience GmbH

<120>  Method for isolating small RNA-molecules using HC-Pro protein

<130>  FB22950

<160>  10

<170>  PatentIn version 3.5

<210>  1
<211>  134
<212>  PRT
<213>  Arabidopsis thaliana

<400>  1

Met Gly Cys Ile Gly Val Val Asn Val Thr Ser Ile Asn Pro Tyr Leu
1               5                   10                  15

Thr Arg Arg Ser Asn Lys Pro Tyr Asn Ser Arg Arg Pro Ser Ser Met
            20                  25                  30

Ala Ala Ala Ile Arg Ala Glu Ser Thr Glu Lys Leu Gly Ile Thr Ile
        35                  40                  45

Glu Lys Asn Pro Pro Glu Ser Lys Leu Thr Gln Leu Gly Val Arg Ser
    50                  55                  60

Trp Pro Lys Trp Gly Cys Pro Pro Ser Lys Phe Pro Trp Thr Tyr Ser
65                  70                  75                  80

Ala Lys Glu Thr Cys Tyr Leu Leu Gln Gly Lys Val Lys Val Tyr Pro
                85                  90                  95

Asn Gly Ser Asp Glu Gly Val Glu Ile Glu Ala Gly Asp Phe Val Val
            100                 105                 110

Phe Pro Lys Gly Met Ser Cys Thr Trp Asp Val Ser Val Ala Val Asp
        115                 120                 125

Lys His Tyr Gln Phe Glu
    130

<210>  2
<211>  750
<212>  DNA
<213>  Arabidopsis thaliana

<400>  2
atatgctata cgattatatt ggacagaaaa tgaaaaattt aaaatggagt tttctattgc        60

attttgtaag ctgagaacaa ctaagttaaa gggaaagcaa aaatgggctg catcggtgtt 120

gtgaatgtaa caagcataaa tccatatctc acaagaagat caaacaaacc ctacaattca 180

agaagaccat catccatggc tgctgcgatt agagctgagt ctactgagaa attagggatt 240

acaattgaaa agaatcctcc tgagtccaaa ctaacccaac tcggtgttcg tagttggccc 300

aagtggggtt gtcctccaag caagtttcca tggacttaca gtgcaaagga gacttgttat 360

ttactacaag gaaaagtgaa agtgtaccct aatggatccg atgaaggcgt agagatcgaa 420

gcaggcgact ttgttgtttt ccctaaagga atgagttgca cttgggatgt ctctgttgct 480

gttgataaac attaccaatt cgagtgaatg gctgctatca atctctgctc tacgtattcg 540

tgtattcgat aggacttgac catctttgta tgttgatgga tacttacttt aagcttgtgt 600

gaagtttttt actttagtgg aaatagagga gtcatatatg gacgcattat ctcattcaac 660

accaaagtca ttcgaagttc gatacaataa catgtatagt gtttactgtc gaaaagtaaa 720

taattgataa tagaagtgat attcacgtaa 750

<210> 3
<211> 36
<212> DNA
<213> zucchini yellow mosaic virus

<400> 3
gctctagagc ggccgcatcg tcacaaccgg aagttc 36

<210> 4
<211> 35
<212> DNA
<213> zucchini yellow mosaic virus

<400> 4
gctctagatt aagcggccgc gccaactctg taatg 35

<210> 5
<211> 19
<212> RNA
<213> artificial sequence

<220>
<223> artificial siRNA duplex

<400> 5
agagugccau gcccgaagg 19

<210> 6
<211> 21
<212> RNA
<213> artificial sequence

<220>
<223> artificial siRNA duplex

<400>  6
agagugccau gcccgaaggu u                                                21

<210>  7
<211>  24
<212>  RNA
<213>  artificial sequence

<220>
<223>  artificial siRNA duplex

<400>  7
agagugccau gcccgaaggu uauu                                             24

<210>  8
<211>  21
<212>  RNA
<213>  artificial sequence

<220>
<223>  ath-miR171a

<400>  8
ugauugagcc gcgccaauau c                                                21

<210>  9
<211>  21
<212>  RNA
<213>  artificial sequence

<220>
<223>  ath-miR171b,c

<400>  9
uugagccgug ccaauaucac g                                                21

<210>  10
<211>  21
<212>  RNA
<213>  artificial sequence

<220>
<223>  ath-miR396a

<400>  10
uuccacagcu uucuugaacu g                                                21

## Description

The present invention relates to a method for isolating small RNA-molecules (sRNAs) from a sample using Helper-component-protein (HC-Pro) from potyviruses. The present invention furthermore relates to an improved method for isolating sRNAs from a sample using HC-Pro together with a cupin protein or the cupin 2-superfamily domain thereof. The methods of the present invention can be used in the treatment, diagnosis and monitoring of diseases, and for screening compounds modifying the number, amount, and/or concentration of sRNAs. Finally, the invention also relates to a kit for isolating sRNAs from a sample.

### Background of the invention

A large part of gene regulation in eukaryotes takes place at the level of RNA-molecules. Currently, a group of endogenous regulatory RNA-molecules, the so-called micro RNAs (miRNAs), is the focus of intensive research. These sRNAs appear to be involved in the regulation of about 30% of all human genes.

In the genome of the respective organism, miRNAs are encoded by miRNA-genes, and their transcription requires the activity of RNA-polymerase II. In mammals, the miRNA-precursors are converted into mature miRNA-molecules through nucleolytic cleavage by the ribonuclease Drosha, and, subsequently, by the ribonuclease Dicer. The mature miRNA-molecules are double-stranded RNA-molecules (dsRNAs) with a size of about 21 base pairs, which typically carry 5'-phosphate residues and overhangs of two nucleotides on both 3'-ends. One of the miRNA-strands is introduced into an enzyme complex with slicer-activity (RNA-Induced Silencing Complex, RISC), and a complementary base pairing leads to a cleavage of the target-RNA, or to a block of the translation (Bartel, 2004; Du and Zamore, 2005). Therefore, a regulation of the activity of genes takes place at the post-transcriptional level.

It was recently found that miRNAs also play a role in the development of diseases, such as cancerous diseases. Thereby, the changes of the miRNA-expression pattern are often highly specific, and this allows for new diagnostic possibilities. Since the miRNA-expression profiles are obviously associated with diagnostic and prognostic factors, they are suitable for the development of respective clinical markers. Today, miRNAs are already extracted from different samples (blood, tissue samples), and studied using specific microchips. In addition, the miRNAs from these materials can be cloned and sequenced. MiRNA-profiles are furthermore used as highly specific markers for the diagnosis, prognosis, monitoring, risk evaluation, and general evaluation of possible therapeutic effects, in particular in the field of personalized medicine.

One possibility to detect miRNA-derived biomarkers consists in the analysis of whole-RNA-fractions (which naturally also contain miRNAs) using microchips. For this, in a first step a whole-RNA purification from the sample (tissue, blood) is performed.

Commonly, triazole is used for the purification, but miRNA-purification kits are also commercially available. Then, an analysis of the whole-RNA is performed on a miRNA-microchip. Today, this service is offered by numerous companies (e.g. Febit, Heidelberg, Germany). Nevertheless, when using this method the danger exists that miRNA species which are present in the sample at low concentrations are further depleted during the purification steps or even get lost, which renders the results of the analysis non-representative and increases the risk to overlook potential biomarkers.

For an identification of yet unknown miRNAs it is required to clone and sequence the sRNAs from a whole-RNA-fraction. Nevertheless, both the cloning and the sequencing efficiencies depend from the modifications of the termini of the miRNAs, and from the sequence thereof (Ebhard et al, 2005, PNAS, 102, 13398-13403). Specific modifications lead to a low cloning efficiency, and thus to a "virtual" under-representation of the respective class of miRNAs.

The potyvirus group (named for its prototypical member, potato virus Y (PVY)) is the largest of the 34 plant virus groups and families currently recognized. Potyviruses (*potyviridae*) are single-stranded RNA-plant viruses and are transmitted mainly by aphids, flies or mites. This group contains at least 180 definitive and possible members (30% of all known plant viruses) which cause significant losses in agricultural, pastural, horticultural and ornamental crops (Ward, C.W. & Shukla, D.D. (1991). Taxonomy of potyviruses: current problems and some solutions. Intervirology 32, 269-296.). The potyviral genomic RNA encoding helper component-proteinase (HC-Pro) is a proteinase isolated from the viral suppressor of silencing region of the potyvirus genomic RNA.

Lakatos et al. (in Lakatos, et al. (2006) Small RNA binding is a common strategy to suppress RNA silencing by several viral suppressors. EMBO J 25, 2768-2780) describe the ability of HC-Pro to bind to sRNAs, and the inhibiting effect of HC-Pro on RNA silencing via binding to siRNAs. Lakatos et al. further mention that, when the HC-Pro-sRNA binding assays were repeated in the presence of *Arabidopsis thaliana* plant extracts, an unidentified plant factor significantly and specifically enhanced the affinity of HC-Pro for all duplexes tested. Nevertheless, this phenomenon was not examined further, nor was such an analysis proposed.

The company New England Biolabs, Inc. sells a p19-based miRNA detection kit (catalogue No. E3312S), which is based on the binding properties of the p19 protein (19 kDa) from the Tombusvirus Carnation Italian Ringspot Virus (CIRV), in order to detect miRNAs that form hybrids with a specific probe. The p19 protein binds 21-23mer dsRNAs with nanomolar affinity in a size dependent and sequence independent manner. The p19 is expressed as an amino terminal fusion of the maltose binding protein (MBP) and a carboxy terminal fusion of the chitin binding domain (CBD). The p19 fusion protein bound to chitin magnetic beads (p19 beads) displayed extended binding properties as the native p19 selectively binds 21 bases long siRNAs whereas the recombinant protein also binds 21-23 bases long siRNAs. Neither the native nor the recombinant p19 bind ssRNA or dsDNA of the same length. Using a ³²P radioactive RNA probe, less than 10 picograms of miRNA can be detected in a million fold excess of unlabeled RNA.

This kit is based on the function of p19, which is different from HC-Pro (Lakatos, et al. (2006). Small RNA binding is a common strategy to suppress RNA silencing by several viral suppressors. EMBO J 25, 2768-2780), and limited to the purification of dsRNA (Lakatos et al. Molecular mechanism of RNA silencing suppression mediated by p19 protein of tombusviruses EMBO Journal (2004) 23, 876 - 884).

Furthermore, there is no teaching in the manual of the kit, to use said kit for the cloning of under-represented sRNAs, as for a testing of the function only increasing amounts of synthetic miR-122a were hybridized to a constant amount of ³²P labeled probe in the presence of a large excess of Jurkat cell RNA, without giving the amount of protein necessary in order to bind the synthetic miR-122a. Apparently, the detection limit also decreases with increasing amounts of background RNA, and the detection limit at 10 pg is rather optimistic. Furthermore, there is no teaching to use the kit for the identification of new clinical markers using cloning and sequencing, in particular in case of "virtually" under-represented sRNAs.

Finally, the genomes of Tombusviruses do not include a HC-Pro protein. *Vice versa,* the genomes of Potyviruses do not include a p19 protein. In addition, both proteins are structurally unrelated. Thus, there is no teaching derivable from the p19-kit to develop a HC-Pro protein-based method for the purification of sRNAs.

The technical difficulties as described above currently compromise a reliable and representative purification of sRNAs from samples, which further also compromises the development of reliable clinical markers.

It is therefore an object of the present invention, to provide an improved method for purifying sRNAs from samples. This method should form the basis for other useful applications based on the improved properties thereof, in particular for cloning and sequencing of new important sRNA-related biomarkers. It shall be particularly avoided that miRNAs which are only present at low concentrations in the samples are lost during the purification because of problems with sensitivity, which could distort the result with respect to (new) miRNA-markers. Other objects and advantages of the present invention will become readily apparent to the person of skill upon studying the following description.

According to a first aspect of the present invention, this object is solved by a method for isolating sRNAs from a sample, comprising the steps of
i) incubating said sample with at least one HC-Pro from potyviruses under conditions allowing for a binding of sRNAs to said at least one HC-Pro, and
ii) isolating said sRNAs from said at least one HC-Pro.

The present invention is based on the surprising finding that the plant-viral protein HC-Pro can be used for a binding of sRNAs of different lengths, with both perfect as well as imperfect duplex structure - thus including miRNA-molecules. Thus, this viral protein can be used in order to enrich and to isolate sRNAs from different samples. The protein will therefore help to improve the problems as faced during the development of clinical markers and therapeutics.

The silencing suppressor protein of the *Potyviridae* family is the multifunctional HC-Pro. HC-Pro has been implicated to act as a dimer / multimer (Wang, R. Y. and Pirone, T. P. (1999) Purification and characterization of turnip mosaic virus helper component protein. Phytopathology. 89, 564-567). It is involved in different steps of the viral life cycle, including insect-vector transmission, cleavage of the polyprotein, RNA-binding through its two RNA-binding domains (Urcuqui-Inchima, S., Maia, I. G., Arruda, P., Haenni, A. L. and Bernardi, F. (2000) Deletion mapping of the potyviral helper component-proteinase reveals two regions involved in RNA binding. Virology. 268, 104-111), viral movement and replication, capsid binding, virus synergism, and suppression of RNA silencing (Kasschau, K. D. and Carrington, J. C. (1998) A counterdefensive strategy of plant viruses: suppression of posttranscriptional gene silencing. Cell. 95, 461-470).

HC-Pro can be schematically divided into an N-terminal region which is involved in the aphid transmission process, a C-terminal region which exhibits protease activity and a central region. The central domain contains several highly conserved motifs which are implicated in genome amplification (IGN motif), synergism with other viruses, and systemic movement (CC/SC motif) (Anandalakshmi, R., Pruss, G. J., Ge, X., Marathe, R., Mallory, A. C., Smith, T. H. and Vance, V. B. (1998) A viral suppressor of gene silencing in plants. Proceedings of the National Academy of Sciences of the United States of America. 95, 13079-13084). The two RNA binding domains (A and B) have been previously described as binding sites for nucleic acids with preference for single-stranded RNA. HC-Pro binds sRNA duplexes, e.g. small interfering RNAs (siRNAs) and microRNAs (miRNAs), rendering these RNAs inactive.

In general, any HC-Pro protein that is derived from a potyvirus can be used for the purposes of the present invention. Preferably, the least one HC-Pro protein is derived from a potyvirus selected from the group of zucchini yellow mosaic virus, tobacco etch virus, sugarcane mosaic virus, hordeum mosaic virus, agropyron mosaic virus, canna yellow streak virus, verbena virus Y, tobacco vein banding mosaic virus, pepper veinal mottle virus, chilli veinal mottle virus, shallot yellow stripe virus, thunberg fritillary virus, lily mottle virus, papaya leaf distortion mosaic virus, barley mild mosaic virus, zantedeschia mild mosaic virus, wheat eqlid mosaic virus, blackberry virus Y, peanut mottle virus, sugarcane mosaic virus, soybean mosaic virus, pennisetum mosaic virus, potato virus Y, sweet potato mild mottle virus, turnip mosaic virus, pepper mottle virus, and bean common mosaic virus. Particularly preferred is the least one HC-Pro protein derived from zucchini yellow mosaic virus (ZYMV).

Many HC-Pro proteins have been described, and their sequences can be taken for the database under the following accession numbers, such as NP_734310.1 for sweet potato feathery mottle virus; ABZ80932.1 for Tobacco etch virus; ABG67923.1 for Sugarcane mosaic virus; YP_003208052.1 for Canna Yellow Streak Virus; YP_001936179.1 for Verbena virus Y; YP_001552413.1 for Tobacco vein banding mosaic virus; YP_002539443.1 for Pepper veinal mottle virus; NP_982336.1 for Chili veinal mottle virus; YP_331415.1 for Shallot yellow stripe virus; YP_254715.1 for Thunberg fritillary virus; NP_945137.1 for Lily mottle virus; NP_871729.1 for Papaya leaf distortion mosaic virus; NP_734441.1 for Barley mild mosaic virus; YP_002321500.1 for Zantedeschia mild mosaic virus; YP_001468089.1 for Wheat eqlid mosaic virus; YP_851200.1 for Blackberry virus Y; ABI97348.1 for Peanut mottle virus; AAW78677.1 for Sugarcane mosaic virus; AAB22819.2 for Soybean mosaic virus; YP_025106.1 for Agropyron mosaic virus; ABI99478.1 for Pennisetum mosaic virus; AAG24786.1 for Potato virus Y; ACS29487.1 for Sweet potato mild mottle virus; ACI14452.1 for Turnip mosaic virus; ACI14450.1 for Tobacco vein banding mosaic virus; ACE80693.1 for Pepper mottle virus; and AAM60816.1 for Bean common mosaic virus.

For the purposes of the present invention, one or several HC-Pro proteins can be used, such as, for example, 1, 2, 3, or 4 different HC-Pro proteins.

With the present invention, it is particularly avoided that miRNAs which are only present at low concentrations or numbers in a sample are lost during the purification because of problems with sensitivity, which could distort the result with respect to (new) miRNA-markers. Until now, no efficient and reliable method was known that allowed for enriching low concentrated miRNAs from a sample. In this way, the information as derived from potential miRNA-markers can be improved. MiRNAs which are present in the samples at only very low concentrations, but could be important as biomarkers, are not lost during the extraction step of the present method. In this way, also very low concentrated miRNAs can be developed into valid biomarkers.

The sRNAs can be "virtually" under-represented in a sample due to the apparent lower number, amount or concentration as detected in or purified from a sample, when compared to other RNA-molecules as detected in or purified from the same or another sample. As an example, a virtual under-representation would be present, if after the purification of two different RNA-molecules from a sample that initially are present in the same (copy) number, amount or concentration, one of the RNA-molecules is detected as having a lower (copy) number, amount or concentration than the other. The under-representation can occur due to problems with the sensitivity of the purification, due to, for example, size differences or modifications, e.g. of the termini of miRNAs, or from the sequence or the strand-form of the RNA-molecules.

Furthermore, the HC-Pro proteins as used in the method according to the present invention can be part of a fusion protein, and are, for example, preferably fused to a fluorescent marker, a histidine-tag (His-tag), a maltose binding protein (MBP), and/or a chitin binding domain (CBD) or may contain deletions that do not negatively interfere with the sRNA binding capacity of the HC-Pro protein. Such deletions may also be introduced in order to improve the sRNA binding capacity of the del-HC-Pro protein, and/or to allow for a more efficient and/or convenient recombinant production of the HC-Pro protein.

For the purposes of the present invention, the HC-Pro proteins as used can be present in any kind of HC-Pro protein containing sample, as long as the composition of said sample does not substantially interfere with the RNA binding activity of the HC-Pro protein. The sample should also not interfere with the stability or other properties of the sRNAs to be bound (e.g. high salt, enzymes as contained, and the like). Preferred is a method according to the present invention, wherein said HC-Pro protein is present in a plant extract, recombinantly produced in a cellular extract, or is HC-Pro protein as such (usually in a buffer, or added in a dry or lyophilized state), in particular isolated, recombinantly produced HC-Pro protein.

In another preferred embodiment of the method according to the present invention, the sample containing sRNAs can be selected from any sample containing sRNAs, such as preferably a biological sample, blood or blood fractions, serum, body fluids, cells, tissue, a saline solution, an aqueous solution, a buffered solution, a sample from a healthy individual, and a sample from a diseased individual, such as, for example, a cancer sample or a sample from an infection or inflammation.

In yet another preferred embodiment of the method according to the present invention, the sRNAs are selected from single stranded or dsRNA, mRNA, siRNA, miRNA, tRNA, rRNA and mixtures or parts thereof, and in particular are selected from siRNA, miRNA, and mixtures thereof. According to the present invention, "small" RNA-molecules preferably have a size of between 10 to 50 nucleotides, preferably of between 15 to 40 nucleotides, more preferably of between 19 to 30 nucleotides, and most preferred of between 21 to 24 nucleotides. The recombinant HC-Pro as analyzed in the context of the present invention preferentially bound 21 nucleotide siRNAs, more than 19 nucleotide and 24 nucleotide siRNAs. Nevertheless, it is assumed that modified MBP:HC-Pro proteins, for example as a fusion protein having a carboxy terminal fusion, such as, for example, of the maltose binding protein (MBP) or deletion mutants HC-Pro proteins, both as described herein will exhibit an improved binding of sRNAs in the range of between 19 nucleotides to 24 nucleotides, preferably of between 21 to 24 nucleotides.

In another important aspect of the present invention, the invention solves the object thereof by providing a method according to the present invention as described above, further comprising the addition of at least one cupin protein. The NCBI protein database (NCBI Reference Sequences: NP_192768 and NM_117098) describes a 134 amino acid unknown and hypothetical protein from *A. thaliana,* which resembles similarity to the protein cupin (the domain thereof). The molecular function of cupin is unknown (see Jim M. Dunwell, Alan Purvis and Sawsan Khuri, Cupins: the most functionally diverse protein superfamily? Phytochemistry Volume 65, Issue 1, January 2004, Pages 7-17). The cupin protein comprises a cupin 2-superfamily domain, such as, for example, the cupin protein according to SEQ ID No. 1 or the cupin 2-superfamily domain thereof, or the equivalent cupin protein of a plant, such as *N*. *benthamiana,* or the cupin 2-superfamily domain thereof to said sample containing sRNAs.

In this aspect of the present invention, the inventors were able to isolate a factor from plant extracts of *A. thaliana,* and to identify it as a protein which specifically improves the binding activity of sRNAs, including miRNAs, to HC-Pro. This protein belongs to a family of polypeptides comprising a cupin 2-superfamily domain, and is therefore termed herein as "cupin protein". The cupin protein as identified in *A. thaliana* is a protein of unknown biological function having a size of about 15 kDa. The cupin protein clearly improves the binding of HC-Pro for sRNAs. An equivalent cupin protein has been identified in *N. benthamiana* extracts.

With this aspect of the present invention, it is particularly avoided that miRNAs which are only present at low concentrations or numbers in a sample are lost during the purification because of problems with sensitivity, which could distort the result with respect to (new) miRNA-markers. Again, these sRNAs can be "virtually" under-represented in a sample due to the apparent lower number, amount or concentration as detected in or purified from a sample, when compared to other RNA-molecules as detected in or purified from the same or another sample. As an example, a virtual under-representation would be present, if after the purification of two different RNA-molecules from a sample that initially are present in the same (copy) number, amount or concentration, one of the RNA-molecules is detected as having a lower (copy) number, amount or concentration than the other. The under-representation can occur due to problems with the sensitivity of the purification, due to, for example, size differences or modifications, e.g. of the termini of miRNAs, or from the sequence or the strand-form of the RNA-molecules.

This aspect provides an even more improved method for enriching low concentrated miRNAs from a sample. Again, in this way, the information as derived from potential miRNA-markers can be improved. MiRNAs which are present in the samples at only very low concentrations, but could be important as biomarkers, are not lost during the extraction step of the present method. In this way, also very low concentrated miRNAs can be developed into valid biomarkers.

For the purposes of the present invention, the cupin protein or the cupin 2-superfamily domain thereof as used can be present in any kind of cupin protein or the cupin 2-superfamily domain thereof containing sample, as long as the composition of said sample does not substantially interfere with the RNA binding activity of the HC-Pro protein and the interaction of the cupin protein or the cupin 2-superfamily domain thereof and the HC-Pro protein. The sample should also not interfere with the stability or other properties of the sRNAs to be bound (e.g. high salt, enzymes as contained, and the like). Preferred is a method according to the present invention, wherein said cupin protein or the cupin 2-superfamily domain thereof is present in a plant extract, for example selected from an *A. thaliana,* or another plant extract, such as an *N. benthamiana* extract or a cupin protein or the cupin 2-superfamily domain thereof containing fraction thereof, is an isolated cupin protein or the cupin 2-superfamily domain thereof, or is an isolated recombinantly produced cupin protein or the cupin 2-superfamily domain thereof, such as, for example, from *A. thaliana,* or *N. benthamiana.*

In the context of the present invention, using a cupin protein, in one example a three-fold stronger binding of HC-Pro could be detected after adding the cupin protein, compared with a GFP-His-protein as a control. Furthermore, the use of 1, 5, and 10 pg of the cupin-protein was shown to have a concentration dependent effect on the binding strength. It was also found that the cupin protein or the cupin 2-superfamily domain thereof seems to have the ability to prevent, improve and/or revert an impaired RNA binding of the HC-Pro protein, for example because of a proteolysis and/or storing of said HC-Pro protein. Without wanting to be bound by theory, the prevention, improvement and/or reversion of an impaired RNA binding of the HC-Pro protein seems to be related to a stabilizing effect of the cupin protein or the cupin 2-superfamily domain thereof of the RNA binding of the HC-Pro protein. In the context of the present invention, the cupin protein or the cupin 2-superfamily domain thereof also improves the RNA binding of the HC-Pro protein, when lower amounts of Hc-Pro as present lead to a binding of the same amount of sRNAs than higher amounts of Hc-Pro without the presence of the cupin protein or the cupin 2-superfamily domain thereof.

In yet another preferred embodiment of the method according to the present invention, said cupin protein or the cupin 2-superfamily domain thereof improves the binding of sRNAs to at least one HC-Pro protein about 3 to 4 fold, compared to a binding of said at least one HC-Pro protein without said cupin protein or the cupin 2-superfamily domain thereof. Thus, using this method, low amounts of sRNAs can be effectively isolated. Furthermore, also lower amounts of HC-Pro protein can be used in order to obtain the same binding as without the addition of the cupin protein or the cupin 2-superfamily domain thereof. Thus, preferably sRNAs, more preferably double stranded sRNAs, are isolated, compared to the overall population of RNA-molecules in a cell.

In another preferred aspect of the method according to the present invention, a fingerprint of sRNAs for a biological sample is generated, comprising the method according to the present invention as herein, and quantifying the sRNAs as isolated from said biological sample.

In the context of the present invention, a fingerprint (also designated a "pattern" or "expression profile") shall mean to comprise the analysis of a representative number of different sRNAs in a sample, a cell or a tissue in order to obtain an overview of the population of the sRNAs in said sample, cell or a tissue. Preferred is the generation of a "specific" fingerprint of sRNAs for said sample, cell or a tissue, i.e. a pattern that is found only for the particular sample, cell or a tissue. Generating said fingerprint comprises analyzing at least one fraction of said sRNAs in said biological sample, such as, for example, siRNA, miRNA, and mixtures thereof. Preferred is the generation of a fingerprint from 2 or more, preferably 10 or more, and most preferred 50 or more sRNAs. Fingerprints can also be generated for fractions according to size, i.e. wherein said sRNAs have a size of between 10 to 50 nucleotides, preferably of between 15 to 40 nucleotides, more preferably of between 19 to 30 nucleotides, and most preferred of between 21 to 24 nucleotides. Of course, combinations of the above selection criteria can also be used to generate a fingerprint of sRNAs for said sample, cell or a tissue, for example a fingerprint for the miRNA-molecules with a size of 21 to 24 nucleotides. As an example, the following literature describes miRNA fingerprinting in the context of cancers; Lu et al. (Lu J, et al MicroRNA expression profiles classify human cancers. Nature. 2005 Jun 9;435(7043):834-8.), and Lehmann et al. (Lehmann U et al. Identification of differentially expressed microRNAs in human male breast cancer BMC Cancer. 2010 Mar 23;10:109.).

In another preferred aspect of the method according to the present invention, said sample containing sRNAs is a biological sample as described herein, wherein said sample is selected from a sample from a healthy individual, and a sample from a diseased individual, such as, for example, a cancerous sample, a sample from an infection, a sample from an inflammation, a sample from a patient suffering from a cardiovascular disease or a sample from a patient suffering from Alzheimer's disease.

Another preferred aspect of the present invention then relates to a method for diagnosing a disease in an individual, comprising the steps of a) isolating sRNAs from a sample derived from an individual suspected to have a disease to be diagnosed using the method according to the present invention as described above, b) determining the number, amount, and/or concentration of at least one sRNA as isolated in step a), and c) comparing the number, amount, and/or concentration of said at least one sRNA as determined in step b) with the number, amount, and/or concentration of said at least one sRNA in a sample derived from a healthy individual.

As above, the sample containing sRNAs can be a biological sample as described herein, wherein said sample is selected from a sample from an individual having or suspected to have a disease or condition related to the number, amount, and/or concentration of said at least one sRNA, such as, for example, a cancerous sample, a sample from an infection, a sample from an inflammation, a sample from a patient suffering from a cardiovascular disease or a sample from a patient suffering from Alzheimer's disease.

Also this aspect of the present invention preferably comprises the steps of generating a fingerprint of sRNAs according to the method according to the present invention, and comparing said fingerprint as generated in step c) of the method as above.

Preferred is the method according to the present invention, further comprising a monitoring of the number, amount, and/or concentration of at least one sRNA, preferably during a treatment of said individual (see also below). In the context of the present invention, a monitoring usually comprises the repeated isolation of said at least one sRNA according to the present invention over time, and performing a diagnosis based on said at least one sRNA as isolated, for example by generating a fingerprint as described herein. Based on changes in the number, amount, and/or concentration of said at least one sRNA and/or the sRNAs as analyzed in said fingerprint, a diagnosis about a disease or condition related to the sRNAs as analyzed can be made. Furthermore, the progress of said disease or condition and/or an effect of a treatment on said disease or condition can be followed (monitored).

Another important aspect of the present invention then relates to the use of the method according to the present invention in the identification of compounds which can be used or developed into therapeutics and medicaments for the treatment (i.e., at least in part, curing and/or ameliorating and/or preventing the clinical symptoms of) diseases or conditions related to sRNAs, preferably diseases that are, at least in part, based on a modification of the number, amount, and/or concentration of at least one sRNA, such as, for example, cancer, infection, inflammation, cardiovascular disease or Alzheimer's disease. Modification (or modifying) shall mean an increase or decrease of the number, amount, and/or concentration of said at least one sRNA as analyzed, compared to a reference sample (preferably a sample derived from a healthy individual, i.e. an individual not suffering and thus, at least in part, lacking the clinical symptoms of the respective disease or condition under analysis and/or treatment).

Another preferred aspect of the present invention thus relates to a method for screening for a compound modifying the number, amount, and/or concentration of at least one sRNA in a cell or tissue, comprising the steps of a) providing a cell or tissue expressing at least one sRNA, b) contacting said cell with at least one candidate compound or mixture of candidate compounds, c) isolating said at least one sRNA from said cell using a method according to the present invention as above, d) determining the number, amount, and/or concentration of said at least one sRNA as isolated in step c), and e) comparing the number, amount, and/or concentration of said at least one sRNA as determined in step d) with the number, amount, and/or concentration of said at least one sRNA in said cell which has not been contacted with said at least one candidate compound or mixture of candidate compounds. The method can be performed *in vivo* or *in vitro.* As cells or tissues, any suitable cell or tissue can be used which express at least one sRNA, such as prokaryotic cells or eukaryotic cells or tissue. The expression can be based on the natural expression of sRNAs in said cell or tissue, or preferably can be, at least in part, based on a recombinant expression of at least one sRNA in said cell or tissue.

Preferred is a method according to the present invention, further comprising the steps of generating a fingerprint of sRNAs according to a method according to the present invention as described above, and comparing said fingerprint as generated in the step e) of said method. Further preferred is a method according to the present invention, further comprising a monitoring of the number, amount, and/or concentration of said at least one sRNA or said fingerprint of sRNAs during a repeated screening, as described herein.

Further preferred is a method according to the present invention, further comprising identifying the compound or mixture of compounds as screened, wherein preferably compounds are identified which specifically modify the number, amount, and/or concentration of only one sRNA as determined. Even further preferred is a method according to the present invention, further comprising the steps of chemically modifying said compound or mixture of compounds as identified, and repeating said screening with said modified compound or mixture of compounds.

This method is suitable for the determination of compounds that can modify the number, amount, and/or concentration of said at least one sRNA and to identify, for example, inhibitors, activators, or modulators of the number, amount, and/or concentration of said at least one sRNA. Also functional modifiers of the sRNAs may be screened and identified, such as antisense oligonucleotides or enzymes interacting with (small) RNA-molecules.

The potentially modifying substance can be any chemical substance or any mixture thereof. For example, it can be a substance of a peptide library, a nucleotide library, preferably an oligonucleotide library, a combinatory library, a cell extract, in particular a plant cell extract, a "small molecular drug", a protein and/or a protein fragment.

The term "contacting" in the present invention means any interaction between the potentially binding substance(s) with the cells or tissue, whereby any of the two components can be independently of each other in a liquid phase, for example in solution, or in suspension, or can be bound to a solid phase, for example, in the form of an essentially planar surface or in the form of particles, pearls or the like. In a preferred embodiment a multitude of different potentially binding substances are immobilized on a solid surface like, for example, on a compound library chip, and the cell or the tissue is subsequently contacted with such a chip.

Measuring of the effect of the compound on the sRNAs of the cell or the tissue can be carried out using well established methods known in the art, and involves, for example, nucleic acid hybridization using labeled oligonucleotides, chip-technology, and fingerprinting as described herein. As a further step at least one compound can be identified (selected), based on the measured modifying activity or on grounds of the sRNAs of the cell or the tissue as analyzed.

The thus selected binding compound is then in a preferred embodiment chemically modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂ OH, SH, NH, CN, aryl, heteroaryl, COH or COOH group.

The thus modified binding compound is then, preferably individually, tested with the method of the present invention. In this step, the effect on the sRNAs of the cell or the tissue as analyzed is measured. If needed, the steps of identifying the compound, modifying the compound, contacting the compound with the cell or tissue in a method of the invention and measuring the effect on the number, amount, and/or concentration of the sRNAs on of the cell or the tissue can be repeated a third or any given number of times as required. The above described method is also termed "directed evolution" since it involves a multitude of steps including modification and selection, whereby modifying compounds are selected in an "evolutionary" process optimizing its capabilities with respect to a particular property, e.g. its modifying activity, its ability to increase or decrease the number, amount, and/or concentration of the sRNA(s) as analyzed.

In a further embodiment of the method of the present invention the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, Band-Aids or pills. Accordingly a further aspect of the present invention is a pharmaceutical composition for the treatment of sRNA(s) related diseases, such as described herein.

Another preferred aspect of the present invention then relates to a kit comprising materials for performing a method according to the present invention as described herein. In a preferred embodiment, said kit comprises a purified Hc-Pro-protein, preferably together with a purified cupin protein or the cupin 2-superfamily domain thereof, optionally together with stabilizers and buffers. In yet another preferred embodiment, said kit comprises an expression vector for a recombinant expression of the HC-Pro protein and/or the cupin protein or the cupin 2-superfamily domain thereof in a cell. Suitable expression systems for cells and/or tissues are known to the person of skill, and described herein. Other preferred kit components are buffers, such as buffers for washing, elution and binding during the method as performed, media for cell culture, protein components, such as, for example, serum albumin or gelatin, and enzymes, such as, for example, RNase inhibitor or proxidases, dyes for labeling, such as, for example, fluorescent labels to be attached or incorporated into nucleic acids, and nucleic acids, such as, for example, oligonucleotides.

In yet another preferred embodiment, said kit comprises at least one HC-Pro protein and/or at least one cupin protein or the cupin 2-superfamily domain thereof as part of a fusion protein, for example, fused to a fluorescent marker, a histidine-tag (His-tag), a maltose binding protein (MBP), and/or a chitin binding domain (CBD).

Another preferred aspect of the present invention then relates to the use of at least one HC-Pro from potyviruses for isolating sRNAs from a sample, preferably in a method according to the present invention as described herein.

Another preferred aspect of the present invention then relates to the use of at least one cupin protein or the cupin 2-superfamily domain thereof for isolating sRNAs from a sample, preferably in a method according to the present invention as described herein, more preferably in combination with the HC-Pro from potyviruses.

Yet another preferred aspect of the present invention relates to a method for treating a disease or condition related to at least one sRNA in a patient, comprising administering a compound as screened according to a method according to the invention or a pharmaceutical composition comprising said compound as screened according to a method according to the invention as above to said patient.

Preferred is a method for treating a disease or condition which is related to at least one sRNA as described herein in a patient, comprising a method for diagnosis according to the invention, and treating said patient based, at least in part, on said diagnosis as described above. The method can preferably also involve a monitoring as described herein.

Preferred is a method for treating a disease or condition according to the present invention, wherein said disease or condition is related to the modification of the number, amount, and/or concentration of said at least one sRNA in said patient.

Exemplary diseases or conditions that are sRNA-related diseases and in particular miRNA-related diseases are cancer, such as breast cancer, Prader-Willi syndrome, cardiovascular disease or Alzheimer's disease, and additional diseases or conditions are described in the literature, for example in Lu et al. (Lu et al. An Analysis of Human MicroRNA and Disease Associations. PLoS ONE. 2008 3(10): e3420), Lu et al. (MicroRNA expression profiles classify human cancers. Nature. 2005 Jun 9;435(7043):834-8), and Lehmann et al. (Lehmann U et al. Identification of differentially expressed microRNAs in human male breast cancer BMC Cancer. 2010 Mar 23;10:109).

Another preferred aspect of the present invention relates to the use of a kit according to the present invention as above in a method according to the present invention as described herein.

The production of recombinant affinity-tagged fusion proteins in *E*. *coli* has become a standard procedure which enables rapid protein purification. However, the choice of expressing vectors, bacterial strains and purification procedures is case dependent. Among the factors driving this choice are expression levels, final yields and protein solubility. Many tags are available to optimize the expression profile and the solubility of the target protein.

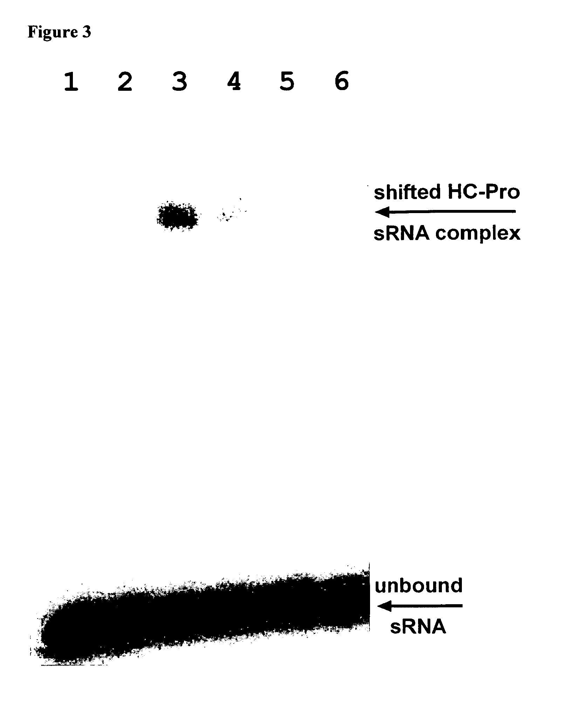

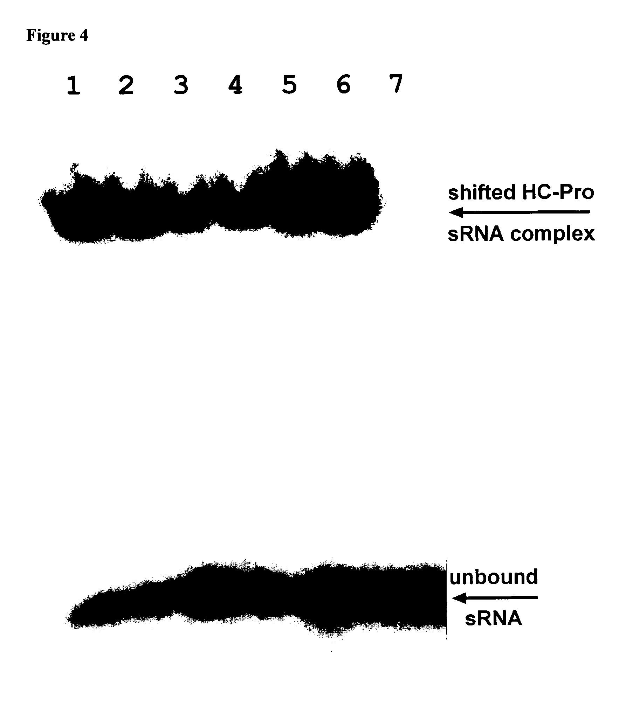

Expressing HC-Pro C-terminally fused to a MBP in the *E*. *coli* BL21 (DE3) *codon plus-strain* at 14°C overnight produced a fraction of partly soluble protein and the purified MBP:HA-HC-Pro bound the 21 bp siRNA (Figures 3 and 4). Binding of siRNA-duplexes by MBP could be excluded, since no RNA binding activity in EMSA was observed using the MBP negative control (Figure 4, lane 1). Thus, it can be concluded that MBP increases the solubility of the HC-Pro fusion protein and does not interfere with the RNA binding activity of HC-Pro.

EMSA using siRNAs with different lengths show that MBP:HA-HC-Pro preferentially binds 21 bp siRNAs (Figure 4) which was also shown for p19 (Vargason, J. M., Szittya, G., Burgyan, J. and Hall, T. M. (2003) Size selective recognition of siRNA by an RNA silencing suppressor. Cell 115, 799-811).

In summary, HC-Pro is a helper component-proteinase which acts as a multifunctional protein in the potyviral life cycle. Apart from its proteolytic activity, HC-Pro has the capacity to bind duplex sRNAs. To investigate HC-Pro-mediated sRNA binding *in vitro,* high amounts of purified protein are required. For this purpose, in the context of the experiments of the present invention as presented in the following, the ZYMV HC-Pro was expressed as a fusion with maltose-binding protein (MBP) in *E. coli.* The expressed fusion protein was purified by affinity chromatography. MBP:HA-HC-Pro was partially soluble. Electrophoretic mobility-shift assays demonstrated that MBP:HA-HC-Pro exhibits sRNA binding activity. The recombinant HC-Pro preferentially bound 21 bp siRNAs more than 19 bp and 24 bp siRNAs. Nevertheless, it is assumed that modified MBP:HA-HC-Pro proteins, for example as a fusion protein similar or even identical to p19 as described herein, will have improved binding of sRNAs over the range of between 19 bp and 24 bp.

Furthermore, in the context of the present invention, the cupin-protein was identified in plant extracts from *A. thaliana* and *N. benthamiana,* which considerably improved the RNA-binding of the HC-Pro protein.

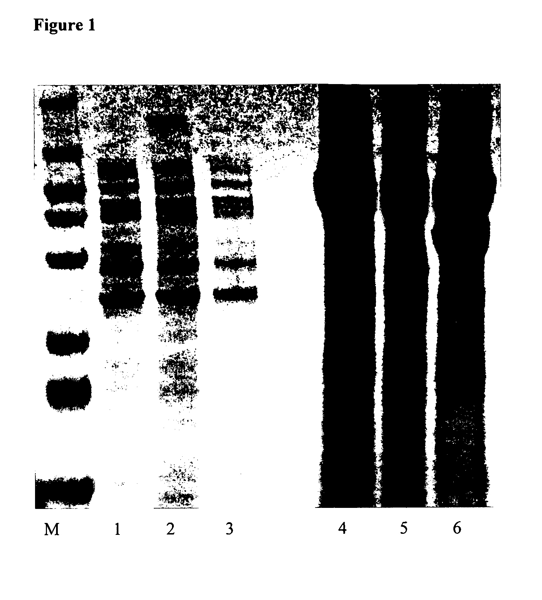

The present invention will now be described in more detail in the following examples with reference to the accompanying Figures and the Sequence Listing, nevertheless without being limited thereto. For the purposes of the present invention, all references as cited herein are incorporated by reference in their entireties. In the Figures
**Figure 1** shows the analysis of the MBP:HA-HC-Pro fusion protein by SDS-PAGE. Coomassie blue stained SDS gel containing samples of soluble (lanes 1 to 3) and pellet fractions (lanes 4 to 6) after expression at 14°C. Lanes 1 and 4 = from BL21 cells; lanes 2 and 5 = from BL21 *codon plus* cells; lanes 3 and 6 = from ArcticExpress cells; M = protein marker, PageRuler (www.fermentas.com).

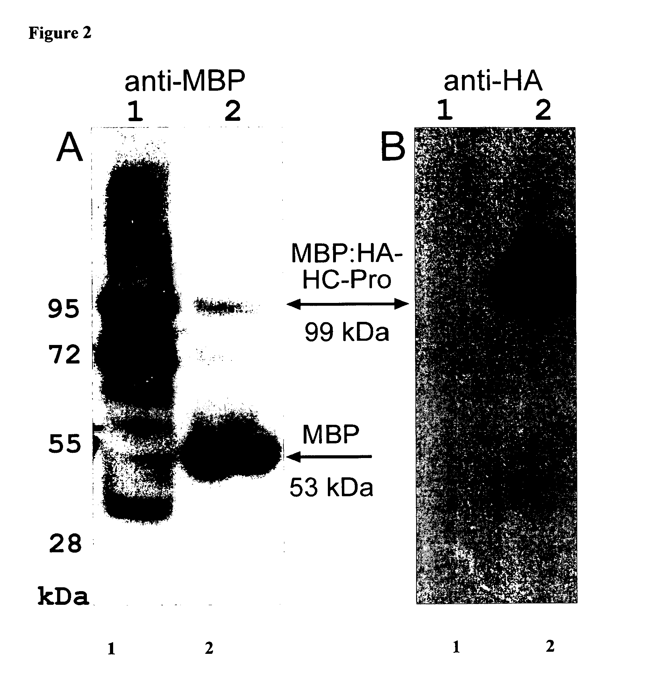

**Figure 2** shows the analysis of affinity purified MBP:HA-HC-Pro. (A) Western blot analysis using an anti-MBP antibody Lane 1 = MBP:HA-HC-Pro from soluble fraction; lane 2 = MBP as a positive control. (B) Western blot analysis using an anti-HA antibody. Lane 1 = MBP as a negative control; lane 2 = bacterial expressed and affinity purified MBP:HA-HC-Pro.
**Figure 3** shows the concentration-dependent binding of a siRNA duplex by MBP:HA-HC-Pro. Electrophoretic mobility-shift assays using a 21nt siRNA duplex and varying amounts of MBP:HA-HC-Pro. Lane 1 = MBP protein; lane 2 = no protein; lane 3 = 800 ng; lane 4 = 400 ng; lane 5 = 200 ng; lane 6 = 100 ng; sRNA = small RNA.
**Figure 4** shows the electrophoretic mobility-shift assays of recombinant MBP:HA-HC-Pro using different sRNA duplexes. Lane 1 and 2 = binding activity using two different 21 bp siRNAs; lanes 3 = binding activity using a 19 bp siRNA; lanes 4 = binding activity using a 24 bp siRNA; lanes 5 = binding activity using ath-miR171b,c; lanes 6 = binding activity using ath-miR396a; lanes 7 = binding activity of MBP (negative control) using a 21 bp siRNA.

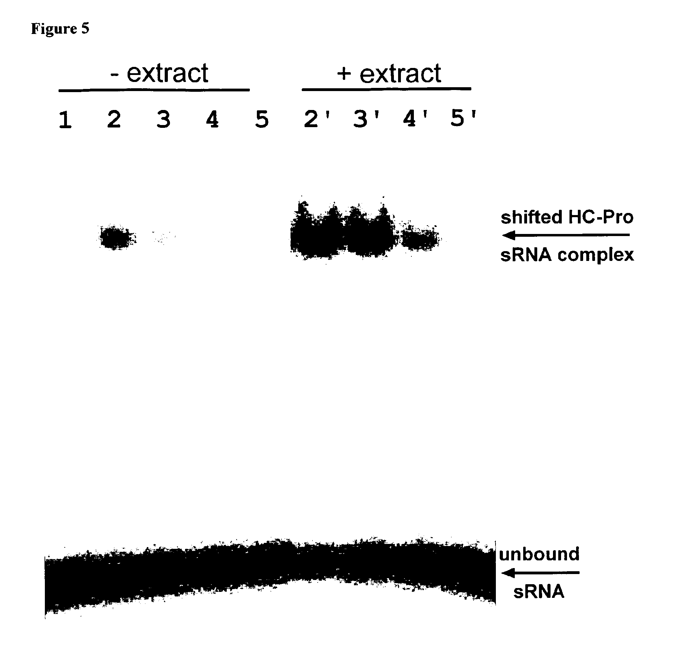

**Figure 5** shows a gelshift-analysis of a concentration dependent binding assays of MBP-HA-HC-Pro-protein from bacteria using a 21 bp siRNA and a plant extract from *A. thaliana; 1* = no siRNA; 2, 2' to 5, 5' = MBH:HA-HC-Pro with concentrations from 800 ng (2, 2') to 400 ng (3, 3') to 200 ng (4, 4') to 100 ng (5, 5').

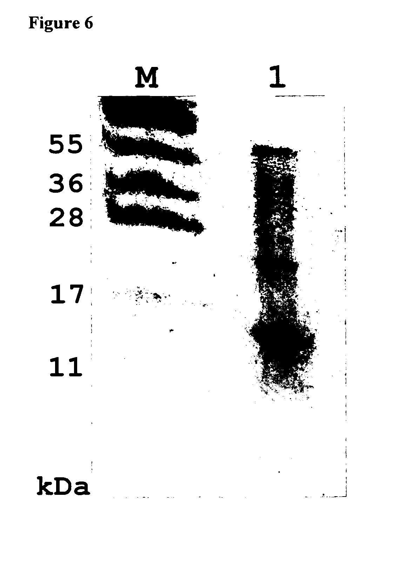

**Figure 6** shows the analysis of *A. thaliana* proteins recovered from a gel filtration chromatography fraction on a silver-stained SDS-PAGE; M = protein marker, PageRuler (www.fermentas.com); 1 = gel filtration chromatography fraction predominantly containing proteins with sizes <20 kDa.

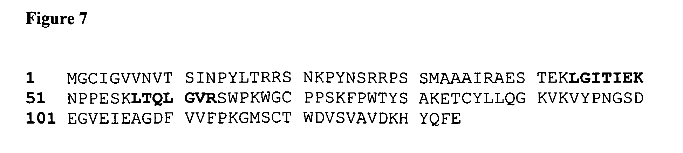

**Figure 7** shows the results of the Maseot-data base analysis with amino acid sequences of the cupin proteins according to the invention. Matching peptide sequences as identified are highlighted in bold.

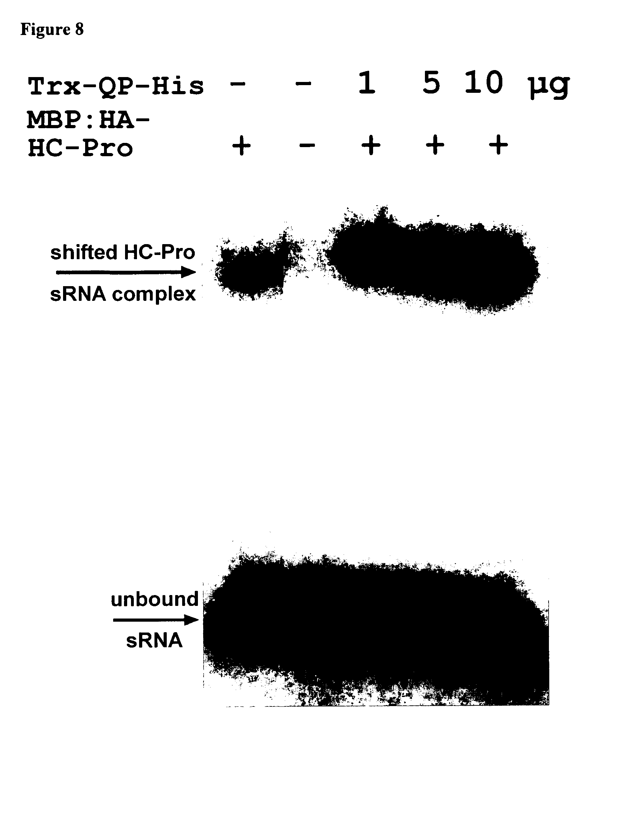

**Figure 8** shows a gelshift-analysis for analyzing the binding of 21 bp siRNA by MBP:HA-HC-Pro using recombinantly expressed Trx-QP-His-protein; + = with MBP:HA-HC-Pro; - = without Trx-QP-His (upper line); - = without MBP:HA-HC-Pro (lower line).

SEQ ID No. 1 shows the amino acid sequence of the cupin protein (database accession number NP_192768).

SEQ ID No. 2 shows the nucleic acid sequence of the cupin protein (database accession number NM_117098).

SEQ ID No. 3 to SEQ ID No. 4 show the nucleic acid sequence of primers as used in the examples.

SEQ ID No. 5 to SEQ ID No. 10 show the nucleic acid sequence of RNA oligonucleotides as used in the examples.

### Examples

### Plasmids

The HA-tagged ZYMV HC-Pro was produced by amplifying the HC-Pro coding region from the aphid non-transmissible ZYMV (ZYMV, GenBank accession no. EF062582) with a 5' primer containing a *Xba*I site and the HA-tag and a 3' primer containing a stop codon and a *Not*I site resulting in pUC57:HA-HC-Pro. For cloning into the pMAL.c2X vector (NEB) the HA-tagged HC-Pro was excised with *Xba*I from the pUC57-HA-HC-Pro plasmid and inserted into the *Xba*I site of pMAL.c2X giving pMAL:HA-HC-Pro. To produce the pET28b(+):His-HC-Pro constructs the HC-Pro was amplified from pUC57-HA-HC-Pro by introduction of two *Xba*I sites in the HC-Pro coding sequence using the HC-Pro fwd primer (5'-GCT CTA GAG CGG CCG CAT CGT CAC AAC CGG AAG TTC-3'; SEQ ID No. 3) and the HC-Pro rev primer (5'-GCT CTA GAT TAA GCG GCC GCG CCA ACT CTG TAA TG-3'; SEQ ID No. 4).

### Protein expression and extraction

*E. coli* BL21 (DE3), BL21 (DE3) *codon plus,* and ArcticExpress cells were transformed with pMAL.c2X and pET28b(+) as negative controls and with pMAL:HA-HC-Pro and pET28b(+):HC-Pro for HC-Pro expression. Cells were grown in LB media containing carbenicillin or kanamycin antibiotics, respectively. At an OD₆₀₀ = 0.6, protein expression was induced by adding isopropyl-β-D-thiogalactopyranoside (IPTG) to a final concentration of 1 mM. After incubation at 37°C for 2 h or at 14°C overnight cells were harvested and resuspended in BugBuster Protein Extraction Reagent (Novagen, 10 ml/g of cells) containing 5 µl Benzonase (25 u/µl); 10 mM DTT and one tablet of complete protease inhibitor (EDTA-free, Roche). The resupended cells were incubated for 1 h at 4°C under agitation. The lysate was centrifuged at 9000 x g for 10 min and the soluble fraction was purified by using amylose magnetic beads or Ni-NTA agarose, respectively, and further analyzed by SDS-PAGE.

### Purification of bacterial expressed MBP:HA-HC-Pro

The bacterial expressed MBP:HA-HC-Pro was purified using amylose magnetic beads (NEB) according to the manufacturer's protocol. 500 µl of amylose beads suspension was washed with MBP buffer (20 mM Tris-Cl, pH 7.4; 200 mM NaCl; 1 mM EDTA; 1 mM DTT) then, beads were added to 10 ml bacterial protein extract and incubated for one hour at 4°C under agitation. A magnetic field was applied and the supernatant was discarded. Beads were washed three times with MBP buffer. For elution of the MBP:HA-HC-Pro 500 µl MBP-buffer containing 10 mM maltose was added to beads and incubated 10 min at 4°C under agitation. The eluted fusion protein was analyzed on a denaturing protein gel.

### SDS-PAGE, Western blot and antibodies

Fusion proteins were analyzed by SDS-PAGE standard methods using 9 % polyacrylamide ready-to-use gels (Anamed). Gels were either stained with Coomassie blue staining reagent or transferred to PVDF membranes using an electrophoresis transfer system (Bio-Rad). Bound antibodies were detected by enhanced chemiluminescence reaction (Pierce). MBP:HA-HC-Pro fusion protein was detected by using monoclonal anti-MBP HRP conjugate (NEB) specific for the MBP or monoclonal anti-HA-POD (Sigma) specific for the HA-tag.

### Electrophoretic mobility shift assays

Labeling and annealing of sRNA duplexes was carried out as previously described with the following modifications. In this study, the inventors used perfect complementary artificial siRNA duplexes with sizes of 19 bp (5'-AGA GUG CCA UGC CCG AAG G-3'; SEQ ID No. 5), 21 bp (5'-AGA GUG CCA UGC CCG AAG GUU-3'; SEQ ID No. 6) and 24 bp (5'-AGA GUG CCA UGC CCG AAG GUU AUU-3'; SEQ ID No. 7). In addition, the artificial miRNA duplexes ath-miR171a (5'-UGA UUG AGC CGC GCC AAU AUC-3'; SEQ ID No. 8), athmiR171b,c (5'-UUG AGC CGU GCC AAU AUC ACG-3'; SEQ ID No. 9) and ath-miR396a (5'-UUC CAC AGC UUU CUU GAA CUG-3'; SEQ ID No. 10) were used.

After annealing of the sRNA duplexes, an RNase protection assay was performed by incubating the samples with 5 units of RNase If (NEB) for 30 min at 37°C, thereby removing non-hybridized single-stranded RNA oligonucleotides. Unincorporated nucleotides were removed with the QIAquick Nucleotide Removal Kit (Qiagen). For binding reactions purified protein and labeled sRNA duplexes were incubated in binding buffer (83 mM Tris-Cl, pH 7.5; 2.5 mM MgCl2; 66 mM KCI; 100 mM NaCl; 0.02 % Tween 20; 10 mM DTT) for 30 min at room temperature. Samples were then electrophoresed at 70 V in 0.5 x TBE buffer using 4 % polyacrylamide gels (Anamed). The gels were dried and quantitative analysis was performed using a Phosphorimager and the PharosFX software (Bio-Rad).

### Expression and Purification of recombinant HC-Pro proteins from E. coli

To increase the solubility of *E. coli*-expressed HC-Pro, it was fused to a maltose binding protein (MBP). It was previously shown that the HC-Pro of potato virus Y (PVY) was partially expressed in a soluble form when it was fused to a MBP. Importantly and similarly to plant-produced HC-Pro, the *E. coli*-expressed protein was capable of binding long RNA (Maia, I. G. and Bernardi, F. (1996) Nucleic acid-binding properties of a bacterially expressed potato virus Y helper component-proteinase. The Journal of General Virology. 77 (Pt 5), 869-877).

Thus, the inventors fused the ZYMV HC-Pro to the C-terminus of MBP using the pMal-c2X vector. The fusion protein could be detected with a MBP-specific antibody. However, in order to exploit a second detectable tag, a HA-tag was introduced between MBP and HC-Pro. The resulting pMAL:HA-HC-Pro plasmid was thereafter transformed into bacterial strains. To optimize soluble protein expression, conditions as were used for pET28b(+):His-HC-Pro expression were applied. Expression of the MBP:HA-+HC-Pro in all three tested *E*. *coli* strains at 37°C resulted in the aggregation of the proteins (Fig. 1). However, expression at 14°C in E. *coli* BL21 (DE3) *codon plus* strain for 16 h produced a soluble form of the protein which was detectable by immunoblot assays using a monoclonal anti-HA and an anti-MBP antibody (Fig. 2). In addition to the full-length protein, some lower molecular mass fragments were immuno-detected, probably representing proteolytic cleavage products of the MBP:HA-HC-Pro. Expression of only the MBP serving as a negative control resulted in a major band at 53 kDa. After affinity purification using amylose magnetic beads the protein was analyzed by SDS-PAGE followed by a Coomassie blue staining and an immunoblot assay. Western blot analysis of the purified MBP:HA-HC-Pro with the anti-HA antibody indicated that the major 99 kDa band consisted of the full-length protein (Fig. 2B).

### Analysis of RNA-binding activity of HC-Pro by EMSA

To determine the minimal concentration of MBP:HA-HC-Pro that would be required for siRNA binding, protein amounts varying from 100-800 ng were used for EMSA. The result showed that MBP:HA-HC-Pro bound siRNAs in a dose-dependent manner. Under the applied conditions, saturation of siRNA binding was reached at a protein concentration of approximately 200-400 ng (Fig. 3).

It was demonstrated that recombinant plant-expressed ZYMV HC-Pro also bound, in addition to 21 bp siRNAs, miRNAs *in vitro* (Shiboleth, Y. M., Haronsky, E., Leibman, D., Arazi, T., Wassenegger, M., Whitham, S. A., Gaba, V. and Gal-On, A. (2007) The inventors investigated MBP:HA-HC-Pro-mediated binding of different sRNA species, including 19 bp, 21 bp, 24 bp siRNAs and a heteroduplex-structured miRNA/miRNA*. The binding activity of HC-Pro could be either sequence or length specific. In order to avoid the sequence specificity, the siRNAs used contained an identical core (19mer) sequence. The result showed that MBP:HA-HC-Pro bound all tested sRNA species although with varying degrees of efficiency (Figure 4).

### Identification of cupin proteins from plants increasing the binding efficiency of sRNA species to HC-Pro

In the context of the present invention, it was analyzed whether HC-Pro from ZYMV exhibited an increased binding of 21 bp siRNA-double strands after the addition of a plant extracts. In order to confirm the effect of an increased binding of the ZYMV HC-Pro-protein after the addition of the plant protein extract, a protein extract from the leaves of *A. thaliana* was produced, and was added in additional gelshift-analyses to the HC-Pro-proteins which were purified by anti-HA agarose.

### Analyses of the binding efficiency of sRNA species to HC-Pro together with cupin proteins

It was examined, to what extent the binding of the MBP-HA-HC-Pro-protein was increased by the addition of the *A. thaliana-protein* extract. In order to study this, different amounts of the HC-Pro-protein (100-800 ng) were used in gelshift-analyses. The result showed that using 800 ng of the HC-Pro-protein without additional factor led to a strong binding of the siRNA-double strand; and that lower amounts of the protein (100-200 ng) did not show binding under the conditions as used. Nevertheless, after the addition of the *A. thaliana*-extract a binding could be already seen when using 100 ng HC-Pro-protein (Figure 5). A comparison of the concentration dependent binding both with and without *A. thaliana-*extract thus led to a clear increase of the binding capacity of the HC-Pro-protein, whereby the binding of 200 ng MBP-HA-HC-Pro with the addition of the extract corresponded to the binding of 800 ng of the protein without extract (Figure 5).

### Purification of the cupin proteins from A. thaliana

Analysis of the *A. thaliana* protein extract which increased the activity of HC-Pro in the gelshift-analyses required a fractionation of the protein extract using gel filtration chromatography with the ÄKTA Prime Plus system, and an analysis using the software *Prime View 5.0.* The column HiLoad 16/60 Superdex 200 pg as used allowed for a separation of the proteins according to their molecular weight in a range of between 10 and 600 kDa.

For this, a protein extract from 10 g of leaves of *A. thaliana* in 5 ml protein extraction buffer was produced, and a chloroform-extraction was performed in order to avoid clogging of the column. The chromatography took place at a constant flow rate of 1 ml/min, and the individual fractions (1 ml each) were collected. Using the UV-detector, a chromatogram could be generated providing information about the chromatographic separation process of the 240 fractions as collected. The peaks were collected into fractions #1 to 10 and analyzed further in gelshift assays using recombinant MBP-HA-HC-Pro-protein from bacteria. The results show that the binding of synthetic 21 bp siRNA-double-strands by 300 ng of the MBP-HA-HC-Pro-protein was increased by the addition of some of the fractions. An increasing effect which was similar to the binding after the addition of the *A. thaliana-protein* extract after chloroform-extraction was particularly found for fractions #7 to 10. Fraction #1, 2, 5 and 6 actually caused a decrease of the binding, which may be explained by the presence of plant proteases or other proteins with unknown adverse functions. For further studies, varying amounts of fractions #7 or #8 were used, which showed similar activities. The further binding-analysis of fraction #7 resulted in a protein band at about 15 kDa after silver staining (Fig. 6).

### Sequence analysis and expression of the cupin protein from A. thaliana

In order to identify the proteins, the sequence data as obtained using mass spectroscopy were analyzed using the Mascot-database. The results as obtained from the database search identified two possible candidates could have an effect on the activity of HC-Pro. Figure 7 depicts the amino acid sequences, and highlights the peptide sequences as determined using mass spectroscopy. The molecular weight (MW) of both proteins was found at between 12 and 15 kDa and thus was in agreement with the MW as detected by silver staining (Fig. 6; 15 kDa band). For a further analysis, the cupin protein from *A. thaliana* was recombinantly produced in bacteria. cDNA was produced from leaves of *A. thaliana-*plants, and using gene-specific primers the encoding sequence was cloned into the bacterial expression plasmid pET32a(+). The pET32a(+)-expression plasmid allows for an expression of the proteins to be examined with an N-terminal thioredoxine-protein (Tm-Tag) (LaVallie et al., 1993), which increased the solubility of the fusion partner, an S-Tag, an enterokinase-cutting site, and a 6 X His-tag both at the N- and C-terminus for a purification of the protein using an Ni-NTA-matrix. The MW of the fusion protein including the tags was 19.1 kDa.

The expression took place in BL21 (DE3) codon plus-cells over night at 14°C. The analysis of the Tm-cupin-protein-His-protein using a Western Blot with anti-his antibody led to strong signals which were found at 34 kDa and corresponded to the MW of the protein.

The detection of the expression of the Trx-cupin-protein-His-protein was achieved by Western-Blot using the anti-his antibody. The amount and the purity of the proteins as isolated were analyzed using a Coomassie-staining of the proteins.

The results show that the Trx-cupin-protein-His-protein was strongly expressed under the expression conditions as used, and could be detected in the Western blot as a signal at 34 kDa. After purification using the His-tag, the protein was present both as a monomer (34 kDa) and as a dimer (68 kDa).

### Gelshift-analyses with the purified protein

In order to detect a possible effect on the binding of siRNA-double strands of MBP-HA-HC-pro-protein, gelshift-analyses with the purified protein were performed. The results show that the addition of 10 pg of the purified Trx-cupin-protein-His-protein led to an increased binding of the 21 bp siRNA-double strands by MBP-HA-HC-Pro (Figure 8), similar amounts of the GFP-His-protein had no significant effect auf die siRNA-binding.

The analysis of the binding was performed using the Quantity One-Software, based on the intensities of the signals of the siRNA-double strands as bound. The binding reaction after the addition of the MBP-buffer served as background value and was subtracted from the other signals. Using this, a three times stronger binding of HC-Pro by the addition of the Trx-cupin-protein-His-protein compared with the GFP-His-protein could be detected.

In order to analyze the concentration dependent effect of the Trx-cupin-protein-His-protein on the activity of the MBP-HC-Pro, lower amounts of the protein were used. The use of 1, 5, and 10 pg of the Trx-cupin-protein-His-protein led to a concentration dependent effect on the binding strength. Furthermore, the Tm-cupin-protein-His-protein did not show binding of the siRNA-double strands (Figure 8).

## Claims

1. A method for isolating sRNAs from a sample, comprising the steps of incubating said sample with at least one Helper-component-protein (HC-Pro) from potyviruses under conditions allowing for a binding of sRNAs to said at least one HC-Pro, and isolating said sRNAs from said at least one HC-Pro.

2. The method according to claim 1, wherein said HC-Pro protein is part of a fusion protein, and is, for example, fused to a fluorescent marker, a histidine-tag (His-tag), a maltose binding protein (MBP), and/or a chitin binding domain (CBD).

3. The method according to any of claims 1 or 2, wherein said sample containing sRNAs is selected from a biological sample, blood or blood fractions, serum, body fluids, cells, tissue, a saline solution, an aqueous solution, a buffered solution, a sample from a healthy individual, and a sample from a diseased individual, such as, for example, a cancer sample or a sample from an infection or inflammation.

4. The method according to any of claims 1 to 3, wherein said sRNAs are selected from single stranded or dsRNA, mRNA, siRNA, miRNA, tRNA, rRNA and mixtures or parts thereof, and in particular are selected from siRNA, miRNA, and mixtures thereof.

5. The method according to any of claims 1 to 4, further comprising the addition of at least one cupin protein comprising a cupin 2-superfamily domain, such as, for example, the cupin protein according to SEQ ID No. 1 or the cupin 2-superfamily domain thereof, or the equivalent cupin protein of a plant, or the cupin 2-superfamily domain thereof wherein said cupin protein or the cupin 2-superfamily domain thereof is preferably present in a plant extract, for example selected from an *A. thaliana,* or a plant extract or a cupin protein or the cupin 2-superfamily domain thereof containing fraction thereof, is an isolated cupin protein or the cupin 2-superfamily domain thereof, or is an isolated recombinantly produced cupin protein or the cupin 2-superfamily domain thereof, such as, for example, from a plant, such as, for example, *A. thaliana.*

6. A method for generating a fingerprint of sRNAs for a biological sample, comprising a method according to any of claims 1 to 5, and quantifying the sRNAs as isolated from said biological sample, wherein said method preferably generates a fingerprint for having a size of between 10 to 50 nucleotides, preferably of between 15 to 40 nucleotides, more preferably of between 19 to 30 nucleotides, and most preferred of between 21 to 24 nucleotides.

7. The method according claim 6, wherein said sample containing sRNAs is a biological sample selected from a sample from a healthy individual, and a sample from a diseased individual, such as, for example, a cancerous sample or a sample from an infection or inflammation.

8. A method for diagnosing a disease in an individual, comprising the steps of:
a) isolating sRNAs from a sample derived from an individual suspected to have a disease to be diagnosed using a method according to any of claims 1 to 5,
b) determining the number, amount, and/or concentration of at least one sRNA as isolated in step a),
c) comparing the number, amount, and/or concentration of said at least one sRNA as determined in step b) with the number, amount, and/or concentration of said at least one sRNA in a sample derived from a healthy individual, and optionally further comprising the steps of generating a fingerprint of sRNAs according to a method according to claim 6 or 7, and comparing said fingerprint as generated, and
d) optionally, further comprising a monitoring of the number, amount, and/or concentration of at least one sRNA, preferably during a treatment of said individual.

9. A method for screening for a compound modifying the number, amount, and/or concentration of at least one sRNA in a cell, comprising the steps of
a) providing a cell expressing at least one sRNA,
b) contacting said cell with at least one candidate compound or mixture of candidate compounds,
c) isolating said at least one sRNA from said cell using a method according to any of claims 1 to 5,
d) determining the number, amount, and/or concentration of said at least one sRNA as isolated in step c), and
e) comparing the number, amount, and/or concentration of said at least one sRNA as determined in step d) with the number, amount, and/or concentration of said at least one sRNA in said cell which has not been contacted with said at least one candidate compound or mixture of candidate compounds, and optionally further comprising the steps of generating a fingerprint of sRNAs according to a method according to claim 6 or 7, and comparing said fingerprint as generated, and
f) optionally, further comprising a monitoring of the number, amount, and/or concentration of said at least one sRNA or said fingerprint of sRNAs during a repeated screening.

10. The method according to claim 9, further comprising identifying the compound or mixture of compounds as screened, wherein preferably compounds are identified which specifically modify the number, amount, and/or concentration of only one sRNA as determined.

11. A kit comprising materials for performing a method according to any of claims 1 to 10, preferably comprising a purified Hc-Pro-protein, preferably together with a purified cupin protein or the cupin 2-superfamily domain thereof, optionally together with stabilizers and buffers.

12. The kit according to claim 11, wherein said HC-Pro protein and/or said cupin protein or the cupin 2-superfamily domain thereof is part of a fusion protein, and is, for example, fused to a fluorescent marker, a histidine-tag (His-tag), a maltose binding protein (MBP), and/or a chitin binding domain (CBD).

13. Use of at least one HC-Pro from potyviruses for isolating sRNAs from a sample in a method according to any of claims 1 to 10.

14. Use of at least one cupin protein or the cupin 2-superfamily domain thereof for isolating sRNAs from a sample in a method according to any of claims 5 to 10.

15. Use of a kit according to claim 11 or 12 in a method according to any of claims 1 to 10.
